# EUROPEAN PATENT APPLICATION

(11) **EP 3 786 288 A1**
(43) Date of publication of application: **03.03.2021**
(21) Application number: 19791928.5
(22) Date of filing: 20.02.2019
(51) Int. Cl.: C12N 5/0784, C12Q 1/02, C12N 15/867, C12N 5/0783, A61K 35/15, A61P 35/00, A61P 31/12

(54) **METHOD FOR EXPANDING HUMAN DC CELL, AND HUMAN DC CELL RESOURCE LIBRARY**

(30) Priority: 23.04.2018 CN 201810368646
(71) Applicant: Celartics Biopharma Co., Ltd, Beijing 100192 (CN)
(72) Inventor: CHENG, Hua, Beijing 100192 (CN); YU, Yang, Beijing 100192 (CN); ZHANG, Huan, Beijing 100192 (CN)
(74) Representative: Glück Kritzenberger Patentanwälte PartGmbB
(86) International application number: PCT/CN2019/075529
(87) International publication number: WO 2019/205783

(57) **Abstract**

Provided is a method for expanding a human DC cell. The method comprises the step of contacting a cell sample of a DC cell to be expanded with a Tax protein sourcing from simian-T-lymphotropic virus (STLV). Also provided are an expanded DC cell prepared by the method, and a DC cell resource library constructed by the method.

## Description

### CROSS-REFERENCE TO RELATED APPLICATIONS

The application claims priority to Chinese Patent Application, filed to the Chinese Patent Office on April 23, 2018, Application No. 201810368646.3, entitled "a method and application for expanding mature and highly active human dendritic cells in vitro".

### FIELD OF THE INVENTION

The present disclosure relates to a method for expanding human dendritic cells (DC), and, especially, a method for expanding DC cells by using STLV Tax protein. The disclosure also relates to a human DC cell resource bank constructed by the method.

### BACKGROUND

Cancer is the number one killer of all mankind and the biggest threat to human health and longevity. China has the largest number of cancer deaths and the fastest increase in tumor incidence in the world. A relevant literature (CA Cancer J Clin 2016; 66(2): 115-32 PMID: 26808342) shows that the number of cancer cases in China in 2015 was 4.29 million, and the death toll was as high as 2.81 million. Immunotherapy is a revolutionary new technology after surgery, radiotherapy, and chemotherapy, and it has shown great potential in anti-cancer therapy. The concept of restoring or enhancing T cell function to achieve tumor killing has been validated. Antibodies targeting immune checkpoints, such as PD1, PDL1, and CTLA4, are effective in clinical therapy. The genetically engineered CAR-T and TCR-T cells have shown amazing anti-cancer effects in certain kinds of cancer or case. However, it has been recognized that therapies with immune checkpoint antibodies or CAR-T and other cell technologies, remain to be modified for improved safety, efficacy and versatility. Issues in treatment, such as immune tolerance, immune escape, etc., remain big concerns in cancer therapy.

Immunotherapy is not only aimed at cancer research, but it also plays a key role in diseases caused by pathogenic viruses. It is well known that, after a virus infects the body, it attacks host cells in an acute or chronic manner, causing inflammation, tissue and organ damage, and even life-threatening consequences in some cases. Some viruses, such as SARS coronavirus, influenza virus, Ebola virus, Zika and dengue fever virus, etc., spread rapidly after infecting the host. Some viral infections are associated with very high mortality and there are no effective preventive or therapeutic drugs for these viral infections. In addition, there are some chronic viral infections, such as hepatitis B virus (HBV), HIV (Human Immunodeficiency Virus, HIV), human papillomavirus (HPV), Epstein-Barr virus (EBV), etc. Long-term infection by these viruses could possibly lead to various types of cancer, and currently there is a lack of curative regimes. Therefore, it is imperative to find an immunotherapeutic method that can combat deadly viral infections.

The immune system is the most important host defense system for recognizing and removing foreign pathogenic microorganisms (such as viruses) and mutated cells (such as tumors) in the body, and the full display of its functions and activities is the key to controlling cancer progression and the aforementioned viral infections. In the immune system, the functional units that play a role are various types of immune cells, including macrophages, B lymphocytes, T lymphocytes, natural killer cells (NK), DC cells, etc. Among them, T lymphocytes, and NK cells mainly mediate cellular immunity, while B lymphocytes prevent the invasion of foreign antigens by producing neutralizing antibodies and mainly participate in humoral immunity, DC cells are the most capable professional antigen presentation cells (APC), participating in both the cellular immunity and humoral immunity, are the key regulators of the entire immune system, and have a huge impact on the function of the immune system.

It is well known that DC cells act as antigen presenting cells (APC), and one of their primary functions is to recognize and present captured antigens to naive T lymphocytes, promote the activation and proliferation of T cells and induce a large number of T lymphocytes that recognize specific antigens and have a strong cytotoxicity, also known as CTL cells (cytotoxic T lymphocytes). Such cells have a direct killing effect on cancer cells and virus-infected cells. Moreover, DC cells have the capacity to activate NK cells. The former can promote the proliferation of NK cells and enhance the activity of NK cells by expressing a variety of membrane proteins and secreting cytokines such as IL12, IL15, and the like. In addition, studies have found that certain cytokines can induce B cells to produce IgG and IgA antibodies, and DC cells can increase the secretion of IgG and IgA after contacting with B cells. DC cells can migrate to the germinal centers of lymph nodes after loaded with antigens, interact with B cells there, and regulate B cell humoral immunity. In conclusion, DC cells regulate the host immune response through a variety of mechanisms and are the one of the most valuable cell types in immunotherapy.

DC-based immunotherapy has been extensively evaluated in clinical trials. These clinical studies provide two aspects of information. First, DC cells are safe to use as cancer therapeutic vaccines. Second, DC cells are effective as cancer vaccines, however the effectiveness is not ideal enough yet. The reason is that the current technologies are difficult to meet the requirements for quantity and activity for the use of DC cell vaccines. First of all, due to the scarcity of primary DC cells and the characteristic of being difficult to culture and expand *in vitro,* it is impossible to directly use them in treatment with adequate amounts of high quality authentic blood DC cells. Therefore, the DC cells used clinically at this stage are a type of cells with DC cell functions differentiated from monocytes after stimulated by a variety of cytokines. However, a large number of clinical data show that such DC cells have poor efficacy. This is because firstly, the number of DC cells is limited, and only about 10⁵-10⁷ cells can be obtained from 100 mL of peripheral blood, which is difficult to acquire an effective therapeutic dose; secondly, the current technology cannot optimize the activity of patient-derived or allogeneic DC cells, which affects the anti-cancer efficacy; and thirdly, the methods of loading tumor antigens through tumor cell lysate treatment or polypeptide transduction of DC cells are inefficient, and the diversity and persistence of effective antigen loading are also limited.

### SUMMARY

In order to overcome the above-mentioned problems, in one aspect, the present disclosure provides a method for expanding DC cells, which comprises contacting a cell sample containing DC cells to be expanded with a Tax protein derived from a simian STLV virus.

In some embodiments, the contacting comprises introducing an expression vector of the Tax protein into the DC cells to be expanded and allowing the Tax protein to be expressed in the DC cells to be expanded.

In some embodiments, the introducing is performed by lentiviral transduction.

In some embodiments, the simian STLV virus is selected from the group consisting of STLV1, STLV2, STLV3, and STLV4 viruses.

In some embodiments, the Tax protein comprises the amino acid sequence as set forth in SEQ ID NO: 1, 2, 3 or 4.

In some embodiments, the cell sample is peripheral blood or cord blood.

In some embodiments, the DC cells to be expanded are obtained by the following steps: a) isolating mononuclear cells from the cell sample; and b) inducing the mononuclear cells to differentiate into DC cells.

In some embodiments, step b) comprises contacting the mononuclear cells with PHA and IL-2 sequentially or simultaneously, or contacting the mononuclear cells with GM-CSF and IL-4.

In some embodiments, the method further comprises introducing an expression vector of a tumor-associated antigen, a tumor-specific antigen or a viral antigen into the expanded DC cells.

In some embodiments, the tumor-associated antigen is hTERT.

In some embodiments, the method further comprises continuing to culture the expanded DC cells in a medium containing IL2 for 3 weeks to 2 months.

In some embodiments, the method does not comprise the step of isolating the DC cells to be expanded from the cell sample.

In some embodiments, the expansion success rate of the method is over 80%.

In another aspect, the present disclosure provides DC cells obtained by the method described above.

In some embodiments, the DC cells are CD11c+ and CD205+.

In some embodiments, the DC cells are CCR7+, HLA-DR+, and CD83, and/or CD40+, CD70+, 4-1BBL+, CD80+, CD83+, CD86+.

In another aspect, the present disclosure provides a method for preparing a cell culture with tumor cell or virus-infected cell killing activity from peripheral blood or cord blood mononuclear cells, which comprises allowing the peripheral blood or cord blood mononuclear cells to be co-cultured with DC cells prepared by the expansion method described above.

In another aspect, the present disclosure provides a cell culture prepared by the method described above.

In some embodiments, the cell culture comprises CTL cells, NK cells, and NKT cells.

In some embodiments, the CTL cells comprise Tα/β cells and Tγ/δ cells.

In another aspect, the present disclosure provides use of the cell culture described above in the preparation of anti-tumor or anti-viral drugs.

In another aspect, the present disclosure provides a method for preparing CTL cells from peripheral blood or cord blood mononuclear cells, which comprises allowing the peripheral blood or cord blood mononuclear cells to be co-cultured with DC cells prepared by the expansion method described above, and isolating CD3+ cells from the co-cultured cells.

In some embodiments, the CTL cells comprise Tα/β cells and Tγ/δ cells.

In another aspect, the present disclosure provides a method for preparing NKT cells from peripheral blood or cord blood mononuclear cells, which comprises allowing the peripheral blood or cord blood mononuclear cells to be co-cultured with DC cells prepared by the expansion method described above, and isolating CD3+ and CD56+ cells from the co-cultured cells.

In another aspect, the present disclosure provides a method for preparing NK cells from peripheral blood or cord blood mononuclear cells, comprising allowing the peripheral blood or cord blood mononuclear cells to be co-cultured with DC cells prepared by the expansion method described above, and isolating CD3- and CD56+ cells from the co-cultured cells.

In another aspect, the present disclosure provides a method for establishing a DC cell resource bank, which comprises respectively obtaining expanded DC cells corresponding to each subject from cell samples of multiple subjects by the expansion method described above.

In some embodiments, the number of subjects is greater than 400.

In some embodiments, the method further comprises detecting and recording the surface marker molecules of the expanded DC cells, the activity of the expanded DC cells, and HLA type of the expanded DC cells.

In some embodiments, the detection of the activity is performed by co-cultivating the expanded DC cells and peripheral blood mononuclear cells, and detecting the killing activity of the obtained cells.

In some embodiments, the method further comprises cryopreserving the expanded DC cells corresponding to each subject separately.

In some embodiments, the method further comprises periodically detecting and recording changes in the activity of the cryopreserved DC cells.

In another aspect, the present disclosure provides a DC cell resource bank prepared by the method described above.

In another aspect, the present disclosure provides a method for preparing a cell culture with tumor or virus killing activity from peripheral blood or cord blood mononuclear cells of a subject, which comprises:
1) detecting the HLA type of the peripheral blood or cord blood mononuclear cells of the subject;
2) selecting DC cells HLA-matched with the peripheral blood or cord blood mononuclear cells of the subject from the DC cell resource bank described above; and
3) Co-culturing of the peripheral blood or cord blood mononuclear cells of the subject and the DC cells.

The DC cell expansion method provided by the present disclosure can efficiently and highly selectively expand DC cells from peripheral blood or cord blood mononuclear cells, and the expanded DC cells have the ability to induce the generation of CTL cells, NK cells and NKT cells from peripheral blood or cord blood mononuclear cells. The human DC cell resource bank provided by the present disclosure can provide highly active DC cell lines for autologous or allogeneic use, which is beneficial for the treatment of tumors or viral infections.

### BRIEF DESCRIPTION OF THE DRAWINGS

Figure 1 is a schematic flowchart for constructing a DC cell resource bank in the present invention.
Figure 2 shows the FACS phenotyping analysis result of a DC cell line (BJ1) in the DC cell resource bank of the present invention.
Figure 3 shows the FACS phenotyping analysis result of another DC cell line (BJ7) in the DC cell resource bank of the present invention.
Figure 4 shows the FACS phenotyping analysis result of T cells produced by inducing a collection of PMBCs with a DC cell line (BJ8) in the DC cell resource bank of the present invention.
Figure 5 shows the results of the killing effect of TERT-specific T cells produced by inducing PBMCs with the BJ8 cell line on osteosarcoma tumor cells. U2OS cells that did not express TERT and U2OS cells that expressed TERT protein were selected to detect the antigen-specific killing effect of T cells. Two hours after the target cell inoculation, T cells were added according to the effector-target ratio (E: T) of 10:1, 5:1, 2:1, and 1:1, and co-cultivated for 4 hours. T cells and dead target cells were aspirated and discarded before the detection.
Figure 6 shows the results of the killing effect of TERT-specific T cells produced by inducing PBMCs with the BJ8 cell line on 3 strains of melanoma target cells. Two hours after the target cell inoculation, T cells were added according to the effector-target ratio (E: T) of 10:1, 5:1, 2:1, and 1:1, and co-cultivated for 4 hours. T cells and dead target cells were aspirated and discarded before the detection.
Figure 7 shows the results of the killing effect of TERT-specific T cells produced by inducing PBMCs with the BJ8 cell line on other 3 strains of target cells. Two hours after the target cell inoculation, T cells were added according to the effector-target ratio (E: T) of 10:1, 5:1, 2:1, and 1:1, and co-cultivated for 4 hours. T cells and dead target cells were aspirated and discarded before the detection.
Figure 8 shows the FACS phenotyping analysis results of CTL, NK and NKT cells produced by inducing PBMCs with a DC cell line (BJ3) in the DC resource bank of the present invention. Both the DC cell line and the PBMCs have an HLA-A2 phenotype.
Figure 9 shows the FACS phenotyping analysis results of CTL, NK and NKT cells produced by inducing PBMCs with a DC cell line (BJ19) in the DC resource bank of the present invention. Both the DC cell line and the PBMCs had an HLA-A2 phenotype.
Figure 10 shows the FACS phenotype analysis results of CTL, NK and NKT cells produced by inducing another collection of PBMCs with a DC cell line (BJ8) in the DC resource bank of the present invention. Both the DC cell line and the PBMCs had an HLA-A0201 and HLA-A2402 phenotype.
Figure 11 shows that a large number of CD56+ cells (NK cells) were sorted out by CD3 magnetic beads for negative selection of immune cells produced by inducing PBMCs with a DC cell line (BJ9) in the DC resource bank of the present invention.
Figure 12 shows the results of the killing activity of NK cells shown in Figure 11 on lung cancer cells A549.
Figure 13 shows the results of the killing activity of the NK cells shown in Figure 11 on mouse NIH3T3 cells expressing the human ligand MICA.

### DETAILED DESCRIPTION

Unless otherwise stated, all technical and scientific terms used herein have the meanings commonly understood by those of ordinary skill in the art.

"DC cells to be expanded" refers to an object to be expanded by the method of the present disclosure, and it may include immature DC cells and mature DC cells. "DC cells to be expanded" may be purified DC cells or DC cells in peripheral blood or cord blood. That is, it is not necessary to separate DC cells from other cells (such as T cells, B cells) before expansion.

"Simian STLV viruses" refer to the form of primate T-cell lymphotropic virus (PTLV) in apes, and are currently only found in a few monkeys and gorillas. In addition to simian viruses (STLVs), PTLV viruses also include human viruses (HTLVs). Both human HTLV viruses and simian STLV viruses have multiple subtypes. Among human HTLV viruses, HTLV1 and HTLV2 are more common. Approximately 5% of people infected with HTLV1 will get sick, including human T-cell leukemia/lymphoma and tropical spastic paralysis. HTLV2 virus infection has little relevance to diseases. The relationship between HTLV3 and HTLV4 virus infection and disease is not yet known. Among simian STLV viruses, 1% of apes infected with STLV1 virus have been found to develop a simian leukemia. The remaining STLV2, STLV3, and STLV4 viruses have not been reported to cause simian diseases, and there is no evidence that they are related to human diseases.

"Tax protein" refers to a protein encoded by the simian STLV virus, which has a domain capable of activating the NF-κB, AP1 and/or STAT signaling pathways, and can promote the expression of viral genes, the transcription of host cellular genes and the transformation of host cells. The inventors unexpectedly discovered that the Tax protein of the simian STLV virus can selectively promote the proliferation and activation of DC cells in mononuclear cells derived from peripheral blood or cord blood, with a success rate of over 80%. In some embodiments of the present disclosure, the Tax protein includes the amino acid sequence shown in SEQ ID NO: 1, 2, 3 or 4.

"Tumor-associated antigen" refers to an antigen that is abnormally highly expressed in tumor cells and has a rather low expression level in normal tissue cells, such as human telomerase reverse transcriptase (hTERT), tumor-testis antigen (CTA), such as MAGE family protein, NY-ESO-1, gp100, GPC3, AFP protein, etc. "Tumor-specific antigen" refers to a protein with amino acid change(s) due to gene mutation(s) in tumor cells, such as hRAS, kRAS, etc.

"HLA typing" refers to the identification of the major histocompatibility complex (MHC) phenotype of a subject or its cells. Methods of HLA typing include traditional serological methods and cytological methods, as well as DNA typing methods developed in recent years, such as PCR-RFLP, PCR-SSO, and so on. Commonly used typing loci include HLA-A, HLA-B, HLA-C, HLA-DR, HLA-DP, HLA-DQ, etc. A "match" of HLA type herein refers to the identity of the alleles of typing loci, involving a match of HLA types between DC cells and peripheral blood mononuclear cells (PBMC) to be co-cultured with the DC cells to induce the production of a cell populations containing CTL cells and a match of HLA types between CTL cells and the cells to be killed by them (such as tumor cells).

A "DC cell resource bank" refers to a cell bank that includes multiple (for example, more than 400, more than 1,000, more than 10,000, or even more than 100,000) separately stored DC cell lines. These cell lines usually originate from healthy individuals. These cell lines can be obtained from the mononuclear cells of the peripheral blood (or cord blood) of healthy individuals using the method of the present disclosure. The method for expanding DC cells provided in the present disclosure can selectively and efficiently expand DC cells from the mononuclear cells of each individual, which is very beneficial to the establishment of such DC cell resource bank. In addition to storing these cell lines, the cell resource bank also keeps other information about each cell line, such as HLA type, DC cell activity, surface markers, quality control data, preservation records, and so on. The DC cell resource bank of the present disclosure is particularly useful for providing HLA-matched active DC cells for allogeneic subjects, which is conducive to solving the clinical problem of HLA matching.

A "subject" refers to a healthy individual, an individual suffering from or suspected of suffering from a certain disease (preferably a human). The term can generally include "healthy volunteer", "patient", "test object", and "treatment object", etc.

In some embodiments, the present disclosure provides a method for expanding DC cells, which involves the use of a Tax protein from a simian STLV virus. As mentioned above, the present inventors discovered that the expression of the Tax protein in a DC cell is able to promote the expansion and activation of the DC cell. The Tax protein may be from STLV1, STLV2, STLV3, or STLV4 virus, and accordingly, may have an amino acid sequence as set forth in SEQ ID NO: 1, 2, 3, or 4. It should be pointed out that, the polypeptides obtained by conservatively substituting one or several amino acids in these sequences, or deleting one or several amino acid residues at the amino or carboxyl end may still have similar functions, so they should also be encompassed within the scope of the present invention. The DC cells to be expanded may be primary DC cells in PBMCs, or immature DC cells and mature DC cells (such as monocyte-derived dendritic cells, MoDC) formed by inducing monocytes in PBMCs. Due to the high selectivity of the method provided in the present disclosure, it is generally unnecessary to perform a DC cell separation step with a cell sample containing the DC cells before the expansion. Coding nucleotides of the Tax protein can be introduced into DC cells in various ways to achieve the expression of the Tax protein in the DC cells. These introduction methods include, for example, liposome- and other positively charged transduction reagent-mediated transduction, electrotransduction, adenovirus or retrovirus-mediated transduction, and the like. In the Examples of the present disclosure, a method for introducing the coding nucleotide sequence of the Tax by lentiviral transduction is provided (see below).

In addition to the introduction of the Tax encoding gene into the expanded DC cells, coding nucleotide sequences of other target proteins can also be introduced, such as tumor-associated antigens or tumor-specific antigens or viral antigens (such as HBV antigens HBsAg, HBcAg, HBxAg, etc.) HCV antigens NS1, NS2, NS3, NS4, NS5, etc.; HPV antigens such as E6, E7, etc.; EBV antigens such as EBNA1, LAMP2, etc.; HTLV virus antigens such as Tax, HBZ, etc.; HIV virus antigens such as Gag, etc.; conserved core antigens of SARS, Ebola, influenza viruses, etc.). DC cells with these antigenic proteins can prime naive T cells to differentiate into CTL cells that are specific for tumor cells or virus-infected cells expressing these target proteins, thereby mediating their antigen-specific killing on target cells.

In some embodiments, the present disclosure provides a method for preparing a cell culture having tumor cell or virus-infected cell killing activity from peripheral blood or cord blood mononuclear cells. The method comprises performing a mixed cell culture of the peripheral blood or cord blood mononuclear cells and the above described DC cells carrying corresponding target proteins (tumor antigens or virus antigens). The cell culture produced form the mixed culture comprises not only CTL cells (including Tα/β cells and Tγ/δ cells), but also NK cells and NKT (natural killer T) cells, which is able to kill tumor cells or virus-infected cells by MHC- restricted and non-MHC-restricted mechanisms. When applied to patients, different mechanisms can act synergistically and it will have a better therapeutic effect.

In some embodiments, the present disclosure provides a method for preparing a DC cell resource bank, which comprises separately obtaining expanded DC cells (DC cell lines) corresponding to each subject from cell samples of a plurality of subjects (usually healthy individuals) by using the DC cell expansion method disclosed herein.

In some embodiments, the present disclosure provides a method for preparing a cell culture with tumor or virus killing activity from peripheral blood mononuclear cells of a subject (usually a tumor patient or a virus-infected patient), which comprises 1) performing a HLA typing of the subject's peripheral blood mononuclear cells; 2) selecting DC cells which are HLA-matched with the subject's peripheral blood mononuclear cell from the DC cell library provided in the present disclosure; and 3) performing a mixed culture with the subject's peripheral blood mononuclear cells and the DC cells. The DC cell resource bank of the present disclosure provides a great convenience for the cell HLA matching in this method.

Particular embodiments of the present invention will be further described in detail below in conjunction with specific operation steps.

### Construction of Virus Vector of Tax Protein and Production of Lentivirus

Coding nucleotide sequences of the Tax protein were introduced into DC cells through lentiviral vectors and expressed in the DC cells

The Tax genes from four STLV viruses were cloned into lentiviral vectors. The amino acid sequence corresponding to the Tax genes were shown in SEQ ID NO: 1 to 4. Synthesis of the nucleotide sequences of the Tax genes were entrusted to a third-party service platform. The nucleotide sequences were inserted into lentiviral vectors, bacterial competent cells were transformed, and after confirmation through sequencing, plasmids were extracted and purified with a plasmid purification kit to obtain high-quality plasmids of the recombinant expression vectors, i.e., Tax plasmids.

293 cells (or 293T cells or 293FT cells) were co-transfected with the obtained Tax plasmids and the packaging plasmids through transduction reagents (such as calcium phosphate transduction reagent, liposome transduction reagent, high molecular polymer transduction reagent, etc.). The packaging plasmids include expression plasmids of VSV-G, Gag-Pol and Rev.

The viruses (hereinafter referred to as Tax lentivirus) could be collected by two centrifugation methods. Method 1: Collect the virus supernatant and centrifuge at 25,000 rpm at 4°C for 2 hours. Method 2: Add PEG8000 and appropriate concentration of NaCl solution to the virus supernatant, and centrifuge at 1600g or 3000rpm for 30-60 min at room temperature or 4°C. After centrifugation, the supernatant was discarded, the virus pellet was resuspended in RPMI1640 medium or other liquid suitable for virus preservation, and then frozen in a deep-low temperature refrigerator (-80°C). The MOI value was measured before storage or before use.

### Construction of Vectors containing tumor or virus proteins and Production of Lentivirus

A tumor or virus antigen gene was cloned into a suitable lentiviral vector to produce a corresponding lentivirus by using a method similar to that for the preparation of the Tax lentiviruses described above.

### Expansion and culture of highly active DC cells

10 mL of peripheral blood (or cord blood) collected from a healthy person or a patient was placed in an anticoagulation tube and sent to a cGMP-level laboratory for mononuclear cell isolation. Here, a density gradient centrifugation method or any known separation methods could be used to separate the mononuclear cells. General steps of density gradient centrifugation for the separation of mononuclear cells were as follows:
1) Transfer the 10 mL blood in the anticoagulation tube to a 50 mL centrifuge tube containing 10 mL of saline or PBS buffer, and mix gently;
2) Take 15 mL Ficoll solution or other similar product solution and add it to another sterile 50 mL centrifuge tube;
3) Gently add 20 mL of the diluted blood to the liquid surface of Ficoll along the wall of the centrifuge tube;
4) Centrifuge at 600 g or 2000 rpm for 20 to 30 min at room temperature;
5) Use a pipette to aspirate the uppermost layer of plasma as much as possible, and the mononuclear cell layer was located at the diluted plasma/Ficoll interface (buffy coat);
6) Collect the buffy coat carefully, add an appropriate amount of saline or PBS buffer to ensure a total volume of 40 mL, and centrifuge at 600g or 2000rpm at room temperature for 10 min;
7) Discard the supernatant, resuspend the cells in 20 mL of saline or PBS, and centrifuge at 600 g or 2000 rpm for 10 min at room temperature;
8) Discard the supernatant, resuspend the cells in a serum-containing medium, and count the cells.

DC cells could be cultured and expanded from the isolated peripheral blood or cord blood mononuclear cells in two ways.

Method 1: expansion of mature and highly active DC cells through primary dendritic cells. The general steps were as follows:
1) Transfer about 2×10⁶∼1×10⁷ fresh peripheral blood or cord blood mononuclear cells to 1 well of a 6-well plate, and add 5 µg/mL Phytohaemagglutinin (PHA) to stimulate and culture the cells (The final concentration of the PHA was usually about 1 µg/mL to about 30 µg/mL);
2) After PHA treatment for about 24 hours, collect the cells, centrifuge and discard the supernatant, resuspend the washed cells in 2 mL RPMI1640 medium, and centrifuge;
3) Discard the supernatant, add 2mL serum-containing PRMI1640 to resuspend the cells and transfer to a new 6-well plate, add IL-2 (final concentration of 50∼200 units/mL) and continue to culture the cells for 2-5 days;
4) Transfer the cell suspension to a 15mL centrifuge tube and centrifuge at 1500rpm for 5 min;
5) Discard the supernatant, resuspend the cells in RPMI1640 medium, and count the cells;
6) Inoculate 2×10⁶ cells in a 6-well plate, add the Tax lentivirus with an MOI of 10 to 50 and 6 to 10 µg/mL polybrene into the well, and culture for 16 to 24 hours;
7) Collect the cells, centrifuge, and discard the supernatant;
8) Resuspend the washed cells in RPMI medium, centrifuge, and discard the supernatant. Resuspend the cells in serum-containing RPMI1640 medium, add IL2 (final concentration of 50∼200 units/mL) and continue to culture the cells;
9) Continue to culture the cells in a medium containing 50-200 units/mL IL2;
10) Continue to culture the cells in a medium containing IL2 for about 3 weeks to 2 months, and analyze the expressions of CD83, CD80, CD86, CD70, CCR7, 4-1BBL, HLA-DR and the like through FACS detection, confirming that a large number of mature and highly active DC cells were obtained.

As an alternative to steps 1) to 3), peripheral blood or cord blood mononuclear cells could also be co-stimulated and cultured in a medium containing PHA and IL2, and then processed with the subsequent steps.

Method 2: Expansion of mature and highly active DC cells through monocyte-derived dendritic cells (MoDC)
1) Transfer about 2×10⁶∼1×10⁷ fresh peripheral blood or cord blood mononuclear cells to 1 well of a 6-well plate;
2) add GM-CSF and IL-4, and culture for 5-7 days;
3) Transfer the cell suspension to a 15 mL centrifuge tube and centrifuge at 1500 rpm for 5 minutes;
4) Discard the supernatant, resuspend the cells in RPMI1640 medium, and count the cells;
5) Inoculate 4×10⁶ cells in a 6-well plate, add the Tax lentivirus with an MOI of 10 to 50 and 5-10 µg/mL polybrene to the well, and co-incubate for 16 hours;
6) Collect the cells, centrifuge, and discard the supernatant;
7) Resuspend the washed cells in RPMI medium, centrifuge, and discard the supernatant; resuspend the cells in serum-containing RPMI1640 medium, add IL2 (50∼200 units/mL) and continue to culture the cells;
8) After the cells were continued to be cultured in a medium containing 50∼200 units/mL IL2 for about 3 weeks to 2 months, the expression of CD83, CD80, CD86, CD70, CCR7, 4-1BBL, HLA-DR and the like was analyzed by FACS, confirming that a large number of mature and highly active dendritic cells were obtained.

### Identification of the immunophenotype of the expanded DCs

The steps are briefly described as follows:
1) Collect DC cells, centrifuge the cells at 2000 rpm for 3 minutes at room temperature, and discard the supernatant;
2) Add PBS-EDTA buffer to wash the cells, pipette the cells up and down gently until there are no clumps, centrifuge the cells at 2000 rpm for 3 minutes, discard the supernatant, and repeat this step one time;
4) Add 100 µL/sample volume of blocking buffer (it may contain BSA or FBS or serum from other sources in a concentration of 0.5%∼3%), flick the cells to mix well, and place it on ice for 10 minutes;
5) Add 1∼5µL of antibody to each sample. After mixing, place it in the dark for 30 minutes, and mix it every 10 minutes to ensure uniform antibody incubation;
6) Centrifuge the cells at 1500rpm for 5minutes, and discard the supernatant;
7) Add 1mL PBS-EDTA buffer to wash the cells, resuspend, centrifuge the cells at 1500 rpm for 5minutes, and discard the supernatant;
8) Add 500 µL PBS to resuspend the cells and detect them with equipment.

### Establishment of a DC cell resource bank

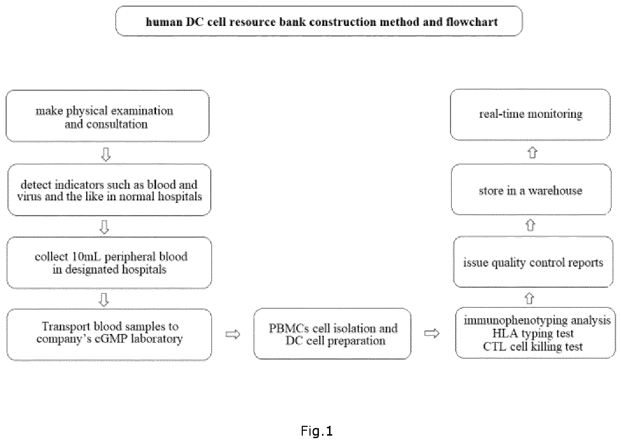

Referring to Fig. 1, a DC cell resource bank including numerous DC cell lines is established by separately expanding DC cells from cell samples (peripheral blood or cord blood) provided by a large number of individuals. The process for establishing the bank is briefly described as follows:
1) Screen individuals who are ready to provide cell samples. The individuals are healthy subjects or early-stage cancer patients and should not have infectious diseases (for example, excluding those infected with hepatitis B virus, hepatitis C virus, HIV, and syphilis). Record the information of these individuals, including birth place, gender, age, physical condition, disease status, etc.;
2) Collect 5∼10 mL of peripheral blood (or 10 mL of cord blood of parturient women during labor) from these individuals and place it in a heparin anticoagulation tube;
3) Separate mononuclear cells from the peripheral blood or cord blood in a GMP-level clean environment;
4) Use the method described above to expand a large number of cells of one group from the mononuclear cells;
5) Perform immune cell phenotype analysis on this group of cells, supplemented by cell morphology observation, etc., to confirm that they are DC cells;
6) Take a part of the DC cells for typing for HLA-A, HLA-B, HLA-C, HLA-DR, HLA-DP, HLA-DQ and the like;
7) Take a part of the DC cells for genetic engineering modification as needed to make them express a desired antigen (such as tumor antigen or viral antigen); co-cultivate the DC cells carrying the antigen and HLA-matched (1 to 12 HLA loci may be matched) peripheral blood or cord blood mononuclear cells to perform Mixed Lymphocyte Reaction (MLR); the obtained cell culture is subjected to flow phenotyping and magnetic bead sorting, and the sorted cells (such as CTL cells, NK cells) are tested for cell killing activity;
8) Cryopreserve the expanded DC cells derived from each individual in at least 4 freezing tubes, with 1∼5×10⁶ DC cells/freezing tube. Perform a sterility inspection and detect mycoplasma, endotoxin, and cell viability on the day of cryopreservation. After passing the quality inspection, the DC cells frozen at -80°C are placed into deep cryogenic liquid nitrogen for long-term storage;
9) Take a part of the frozen DC cells at regular time, thaw them, and test the survival rate of cells after cryopreservation and recovery; expand the cells to detect whether the immune phenotype of the DC cells has changed, and whether the activity of stimulating immune cells has changed;
10) Summarize and record the immunophenotyping data, HLA typing test data, cell killing activity and quality control related data of each DC cell line to form a file of the DC cells and enter it into an information management system.

### Use DC cells in the DC resource bank to induce peripheral blood or cord blood mononuclear cells to produce CTL and NK cells

Active DC cells in the resource bank are co-cultivated with PBMCs (or cord blood mononuclear cells) from tumor or virus-infected patients or healthy volunteers to perform a mixed lymphocyte reaction to produce CTL and NK cells.

The steps are briefly described as follows:
1) Detect the HLA phenotype of mononuclear cells;
2) Take cells that have at least one HLA-A match with the mononuclear cells from the DC cell resource bank, resuscitate and culture them for use;
3) After resuspend the mononuclear cells in a culture medium, count them, adjust the cell concentration to 2∼5×10⁶/well, and place them in a 6-well plate;
4) Take DC cells which are HLA-matched with the mononuclear cells and count them. Add an appropriate amount of DC cells to the wells according to a mononuclear cell: DC cell ratio of 500:1 to 100:1. Place the 6-well plate in a 37°C, 5% CO₂ incubator to culture the cells overnight;
5) Add recombinant IL2 solution to the 6-well plate with a concentration of 100∼500 units/mL the next day;
6) Continue the culture until all DC cells disappear. Take some cells and analyze the types of the induced immune cells and the expression profile of molecules related to immunotherapeutic effect by flow cytometry. The operation method is similar to the above method for the identification of DC cell immune phenotype.

### Determination of the killing effect of CTL cells or NK cells induced by DC cells on target cells

The steps are briefly described as follows:
1) Continue to culture the mixture of NK cells and CTL cells produced by the steps described above to a certain quantity.
2) Collect the cells, centrifuge at 2500 rpm for 5 minutes in the centrifuge tube, and discard the supernatant;
3) Resuspend the washed cells in 5mL PBS, centrifuge at 2500rpm for 5minutes, and discard the supernatant;
4) Resuspend and disperse the cells in 0.5mL medium, add 100µL CD3 magnetic beads, flick and mix;
5) Place the centrifuge tube on a shaker and shake it slowly at room temperature for 15 minutes to make the magnetic beads and cells fully combined;
6) Add 5 mL PBS to the centrifuge tube and mix by inversion. Place the centrifuge tube on a magnetic stand and let it stand for 2 minutes;
7) Aspirate the cell suspension from the side away from the magnetic beads with a pipette, and transfer all of it to a 50 mL centrifuge tube;
8) Remove the centrifuge tube from the magnetic stand and resuspend the magnetic beads with 5 mL PBS. Place the centrifuge tube on the magnetic stand and let it stand for 2 minutes.
9) Aspirate the cell suspension from the side away from the magnetic beads with a pipette, and transfer all of it to the 50 mL centrifuge tube;
10) Repeat the steps 8) and 9);
11) Centrifuge the cells in the 50 mL centrifuge tube, and discard the supernatant. Resuspend the cells in 5mL culture medium, add 100∼500 units/mL IL2, transfer to a culture flask and continue the culture to obtain NK cells;
12) In addition, culture CTL cells after the cells adsorbed by the magnetic beads are resuspended in a culture medium;
13) Resuscitate target cells during the above steps and subculture;
14) After continue to culture NK cells or CTL cells for 3∼5 days, take the target cells (transfected to express luciferase), count them, inoculate 2.5∼10×10⁴ cells/well in a 24-well plate and place in an incubator to culture for 2 hours;
15) Take growing NK or CTL cells, count them, add them to the target cell wells according to an effector-target ratio of 10:1, 5:1, 2:1, or 1:1, and co-cultivate for 4 hours.
16) After 4 hours, aspirate and discard the medium supernatant. Wash the cells in the wells with PBS until all suspended cells are removed;
17) Add 100∼200µL of lysis buffer to the wells to lyse the target cells;
18) Collect the lysed samples of each group and centrifuge at 12000rpm for 1 minute;
19) Take 20µL of supernatant to a 96-well plate, and then add 100µL of luciferase substrate solution to the well.
20) Detect the luciferase activity with equipment and calculate the killing activity.

### Example 1. Phenotype identification of expanded DC cells

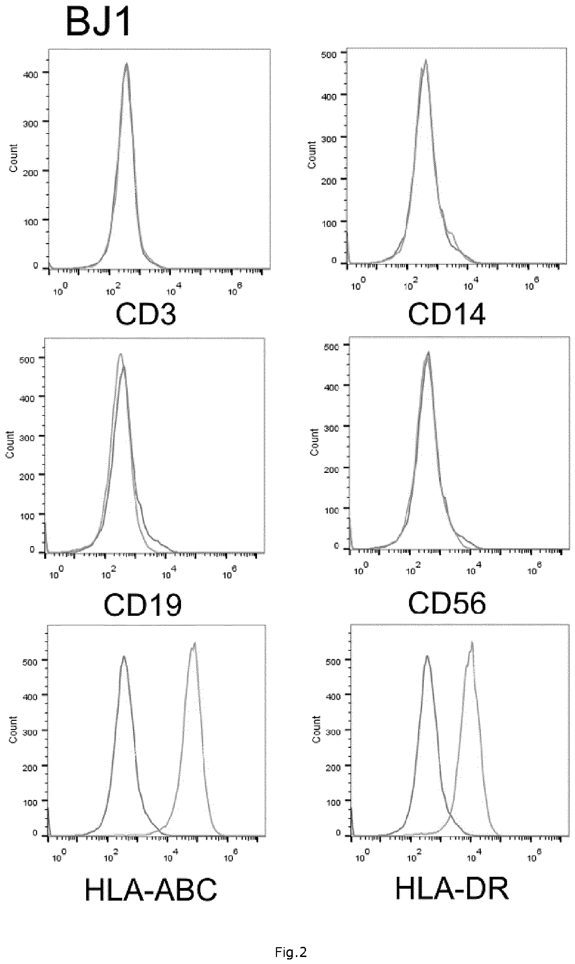

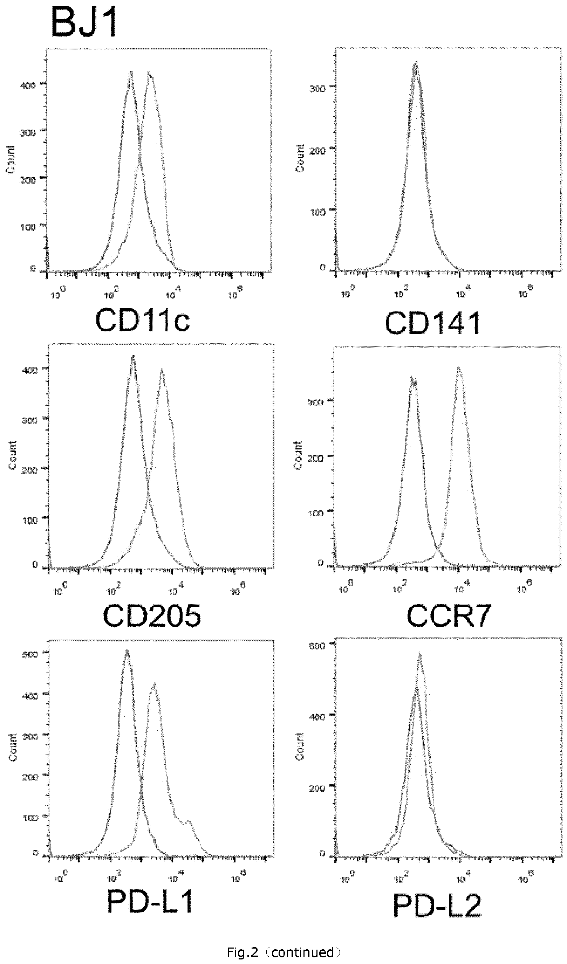

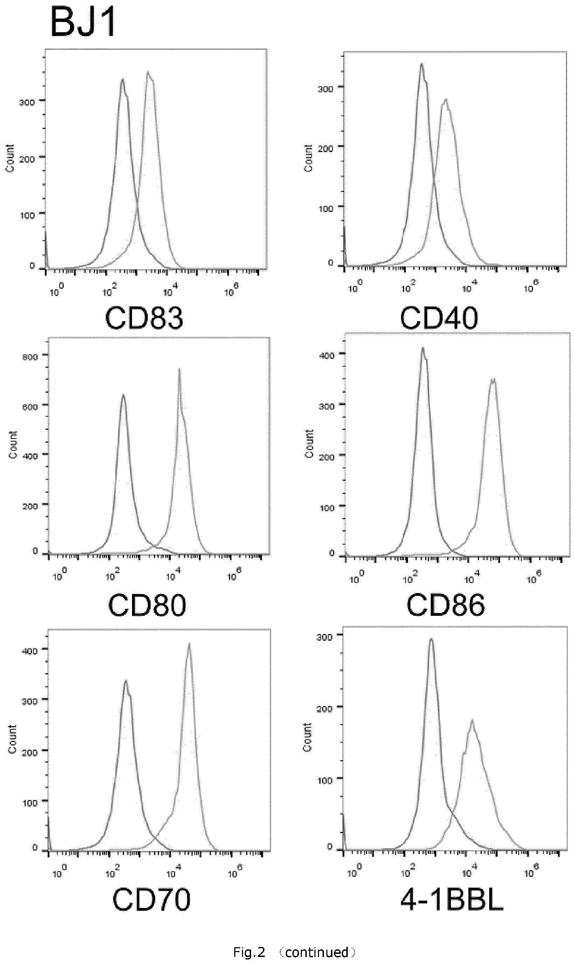

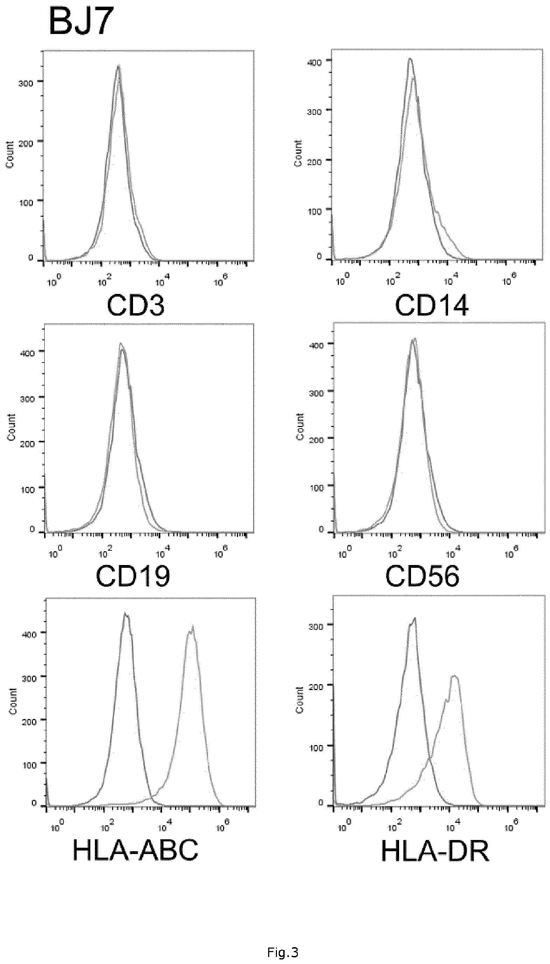

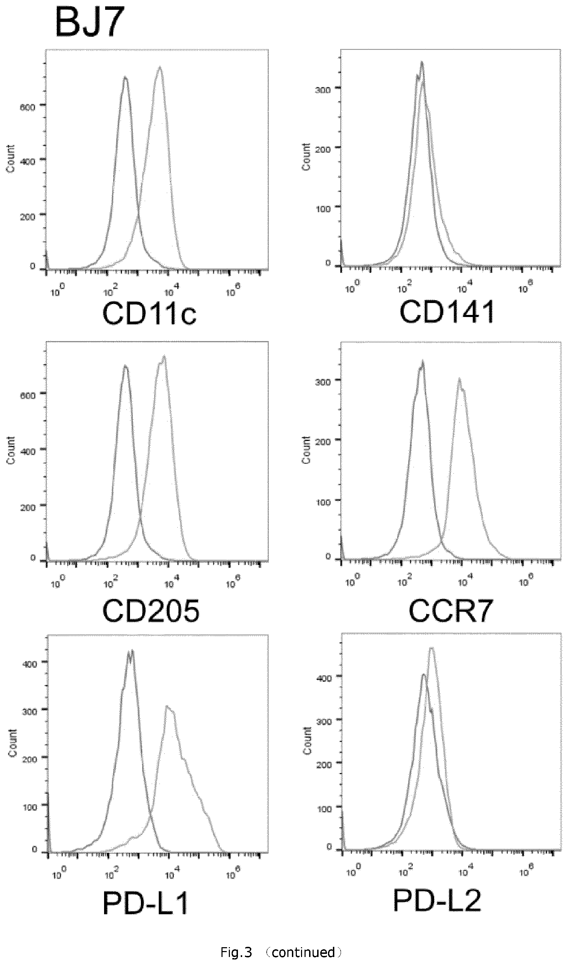

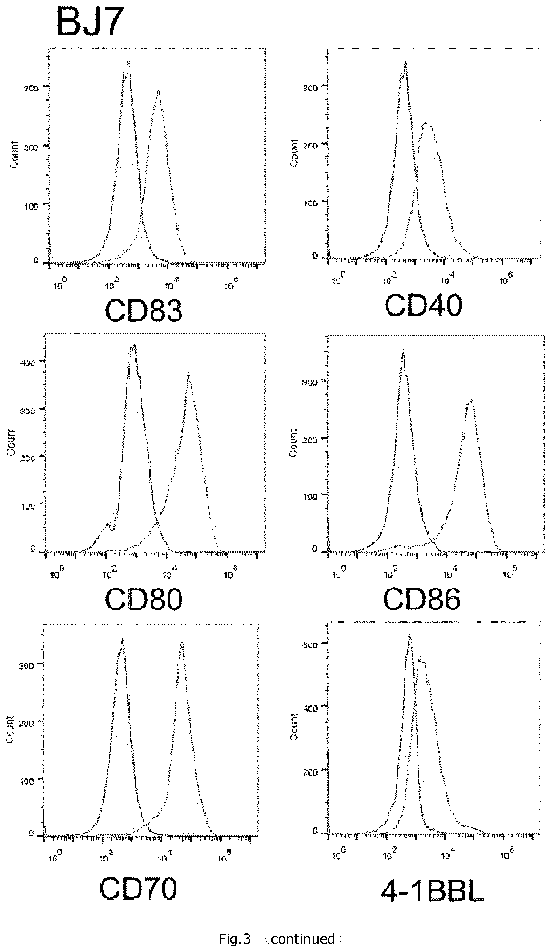

We enriched and attained a large number of cells from each collection of mononuclear cells through the methods described above, including preparation of lentivirus containing Tax gene, isolation of mononuclear cells, preliminary stimulation (cytokine induction) and lentiviral transduction, subsequent culture and screening processes. These cells were subjected to immunophenotyping analysis by flow cytometry, and it was found that they expressed DC cell-related marker molecules. Figures 2 and 3 showed the FACS detection results of cells in the DC cell resource bank obtained after expansion and culture of two collections of PBMCs from different individuals (BJ1 and BJ7). These cells were CD3-negative, CD14-negative, CD19-negative, CD56-negative, and the possibility that they were T cells, monocytes, B cells, or NK cells was ruled out; these cells expressed DC cell-related marker molecules CD11c and CD205; these cells expressed CCR7 , HLA-DR, CD83 and other marker molecules related to DC cell maturation; at the same time, these cells also expressed CD40, CD70, 4-1BBL, CD80, CD83, CD86 and other marker molecules related to DC cell activation. In addition, it could be observed with a microscope that some single suspended cells had many dendrites on the surfaces; during the culture, some cells had the characteristics of adherent growth and showed the morphology of classic DC cells. Therefore, combining the analysis of a variety of immunophenotypic molecules and morphological observation, it was determined that these cells were DC cells.

### Example 2. The method of the present invention expanded DC cells with a high success rate and a high selectivity

We obtained 6 collections of peripheral blood from healthy adults through volunteer donation, each with 7 to 9 mL. The time period from blood collection to the start of experimental treatment was controlled within 3 hours. The 6 blood samples with different individuals were subjected to PBMC separation by the method for separating PBMCs described above. After the cells of buffy coat were obtained, they were washed with PBS, resuspended in a serum-containing medium, and PHA factor was added to stimulate and culture the cells. Starting on the second day, 200 units/mL (final concentration) of IL2 protein was added to the cells for culture. After 3 days of stimulation and culture, the stimulated cells were transfected with the STLV3 Tax lentivirus prepared earlier, and IL2 was added to continue the culture. Afterwards, change or add IL2-containing medium according to the cell growth status and continue the culture. After 3-4 weeks of culture, some cells would grow slowly and die gradually, and the remaining cells would continue to be cultured. After two months of continuous culture, we collected some cells for flow cytometry analysis and morphological observation (as described in Example 1), which confirmed that the cells that continued to grow were DC cells. Among the 6 peripheral blood samples, one sample gradually died after being cultured for a period of time, while the remaining cells of 5 samples were cultured for more than 4 months. After culturing for 2 months and confirming that they were DC cells through flow cytometry, we began to cryopreserve some of the continuously expanding cells, and continued to culture the remaining cells. Later, we resuscitated the cryopreserved cells and cultured them, and found that they still maintained good growth and expansion capabilities. Therefore, the method of the present invention successfully expanded active DC cells from 5 out of 6 peripheral blood samples (83% success rate). We judged that the expansion failure of the other one of the samples might be caused by an operational error. To confirm this hypothesis, we found the donor of the blood sample that failed to expand and asked for another donation of 8 mL of peripheral blood. The method of the present invention was used to carry out the culture and expansion again, and after a culture period of at least 4 months or more, the expansion and activation were successful. Therefore, technically speaking, the DC cell expansion method provided by the present disclosure can efficiently achieve the expansion and activation of DC cells, and the success rate is close to 100%.

On the other hand, it is known that DC cell account for only 1% of the number of PBMC cells, T cells account for about 58%, B cells account for about 20%, NK cells and monocytes account for about 10%, and NKT cells account for about 1%.

On the other hand, it is known that DC cell account for only 1% of the number of PBMC cells, T cells account for about 58%, B cells account for about 20%, NK cells and monocytes each account for about 10%, and NKT cells account for about 1%. We obtained only 7-9 mL of peripheral blood from healthy volunteers. After separation, the obtained 0.8-1.5×10⁷ PBMC cells were all used for the expansion of dendritic cells. We did not detect the content of DC cells in these cells or separate DC Cells or monocytes from these cells. During the culture, there would be two groups of cells: cells of one group were easy to aggregate into a clump to grow, and cells of the other group were individually scattered in the medium. It was found by continuous observation that after 3 to 5 weeks of culture, the number of cells growing in clumps would increase, while individual scattered cells gradually disappeared. Therefore, after culturing for 2 months according to the method of the present invention, the cells we obtained were basically clumped cells. Without any sorting treatment, it was proved by various marker molecules and morphology that these cells were pure DC cells. This is especially important for allogeneic use to prevent other potentially contaminated cells, such as T cells, from causing graft-versus-host disease.

### Example 3. The ability of expanded DC cells to induce antigen-specific T cells

Telomeres in normal cells progressively shorten as cells divide. When they reach a certain level, the cells will age and die. Activation of the telomerase is able to keep the lengths of telomeres relatively stable, thereby enabling cells to gain the ability to proliferate indefinitely and further develop carcinogenesis. Therefore, the abnormality of telomerase activity is closely related to the occurrence and development of tumors. Telomerase is composed of telomerase reverse transcriptase (hTERT), telomerase RNA and a pseudouridine synthase, and hTERT is a restrictive component of the telomerase activity. Studies have found that hTERT is highly expressed in more than 90% of all types of tumor and plays an important role in tumorigenesis and cancer development. Because it is almost not expressed in most normal tissue cells, hTERT is a very ideal tumor target.

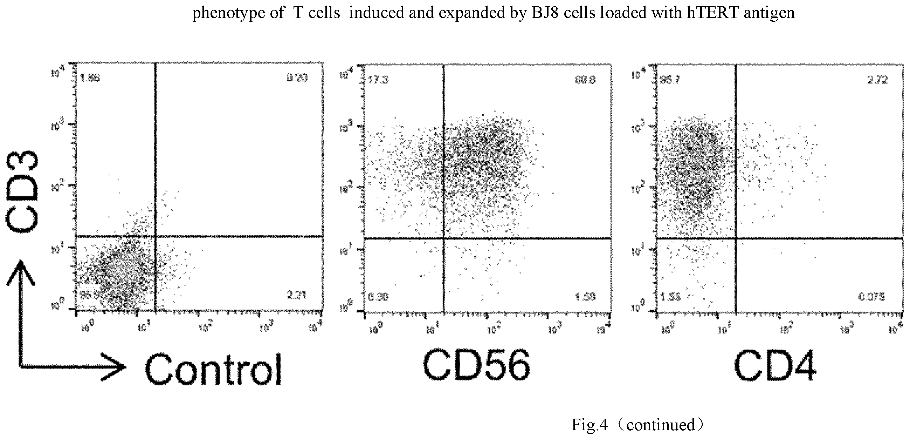

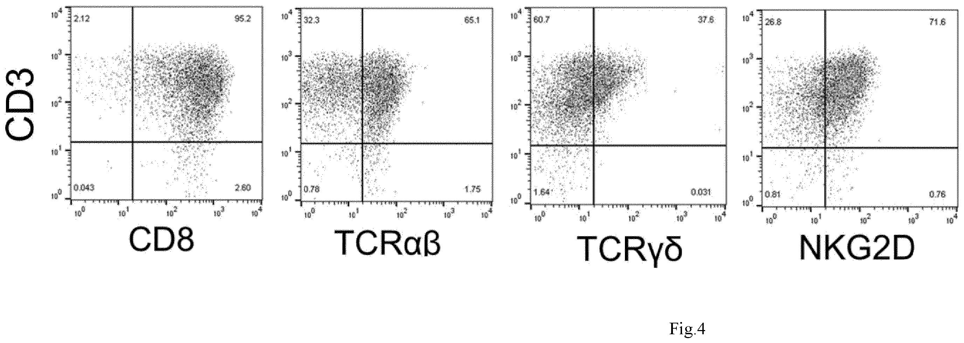

Based on this, in this example, a DC cell line (BJ8, established after transduction with STLV4 Tax protein, HLA phenotypes A0201+ and A2402+) was selected from the DC cell resource bank, and transduced with lentivirus to continuously express hTERT (DC -hTERT). PBMCs, which were HLA-A matched with the DC-hTERT cells, were selected and subject to MLR test to co-culture and stimulate the production of a large number of immune cells. The latter was sorted by CD3 magnetic beads to obtain a separate group of cells. This group of T cells were mainly CD8+ T cells, as observed by flow cytometry (Figure 4); according to the type of TCR receptors, it contained Tα/β cells and also contained Tγ/δ cells. Tα/β cells kill target cells in an MHC-restricted manner, while Tγ/δ cells kill cells in a non-MHC-restricted manner and may act as coordinated attackers. At the same time, some cells expressed CD56 molecules and NK cell activation related receptor NKG2D, suggesting that this line of DC cells was able to induce the production of NKT-like cells while stimulating the expansion of CTL cells.

### Example 4. Killing effect of hTERT-specific T cells induced by expanded DC cells on tumor

In this example, HLA-A0201+/A2402+ PBMC cells were co-cultivated with the parental DC cells and the DC-hTERT of Example 3 to produce T cells. These two types of T cells were tested for their killing effect on tumor cells after sorted out by CD3 magnetic beads.

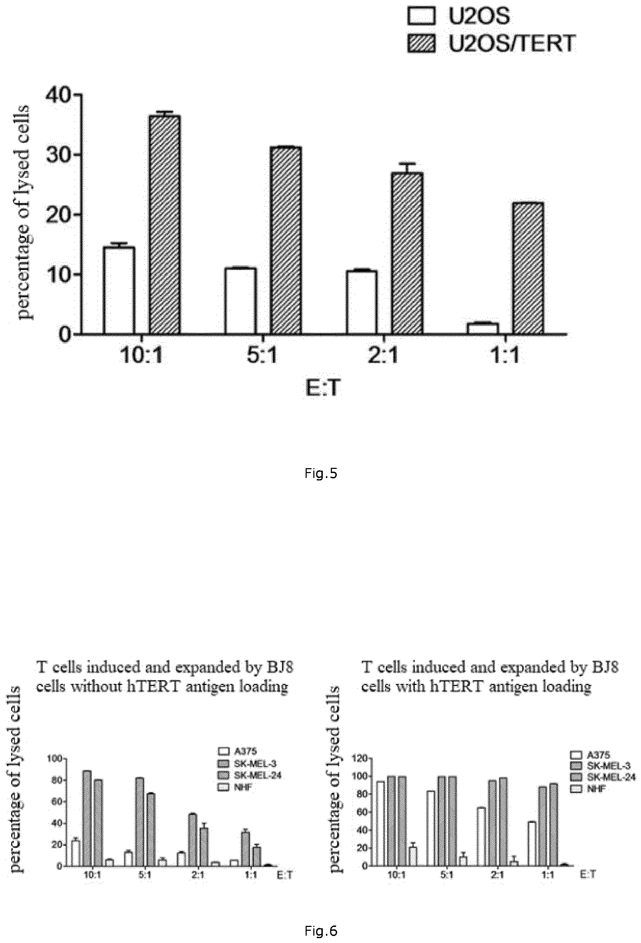

First, this study needed to confirm that the T cell killing was hTERT specific. Therefore, in this study, human osteosarcoma tumor cell U2OS (HLA-A2) was choose as target cells. A major feature of this target cells was that the background expression of TERT protein was very low, almost undetectable. U2OS/TERT modified by genetic engineering could express hTERT protein. The study selected the parental U2OS and U2OS/TERT as target cells to detect the killing effect of hTERT-specific T cells. It could be seen from Figure 5 that for the parental U2OS cells that did not express TERT protein hTERT-specific T cells have a weak killing effect on them. The slight killing at a high effector-target ratio (10:1) might be caused by Tγδ cells in T cells. The killing effect of T cells on U2OS cells was significantly enhanced after U2OS cells expressed TERT protein, suggesting that this method induced hTERT-specific T cells.

Next, three melanoma cell lines were used as target cells, and normal human fibroblast NHF (HLA-A2/A2) was used as a control (Figure 6). The three tumor cell lines were A375 (HLA-A0101/A0201), SK-MEL-3 (HLA-A2402) and SK-MEL-24 (HLA-A1/A2). All of these three DC cell lines had only one HLA matched with T cell HLA. From the results shown in Figure 6, it could be seen that A375 cells were not sensitive to the T cells that were not specifically directed to the TERT antigen, and the T cell killing effect was only 20% under the condition of an effector-target ratio of 10:1; while the TERT-specific T cells had a killing effect of more than 90% on A375 tumor cells. Even under the condition of an effector-target ratio of 1:1, the killing ability of the hTERT-specific T cells was over 50% on A375 cancer cells. For both SK-MEL-3 and SK-MEL-24 cell lines, the killing effect of non-specific T cells was less than 40% when the effector-target ratio was 1:1, while the hTERT-specific T cells had a killing effect of more than 90% at an effector-target ratio of 1:1, suggesting that the hTERT-specific T cells could generate targeted killing of TERT-expressing tumor cells at a lower effector-target ratio. Moreover, the killing effect of hTERT-specific T cells on NHF was very weak, suggesting that the TERT-specific T cells could selectively kill tumors and had high safety profile.

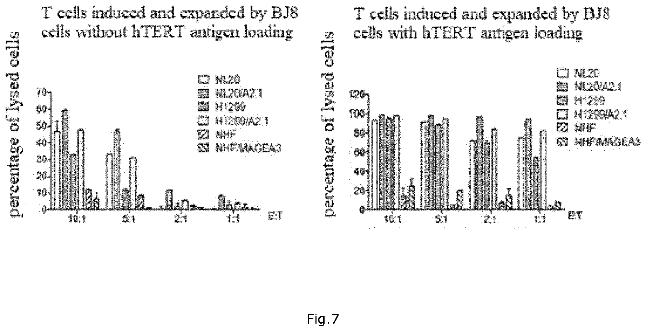

In another study, three cell lines of NL20 cells (bronchial epidermal cells that could proliferate infinitely after genetic modification, HLA-A1/3), H1299 (lung cancer cells, HLA-A32/A24) and normal human fibroblasts (NHF, HLA-A2/A2) were choose as research objects. First, the three cell lines were genetically modified to allow NL20 cells express HLA-A0201 molecules, which could be recognized by T cells with the same HLA type; to allow H1299 cells express HLA-A0201 molecules, so that the cells were matched with T cells with the same HLA-type (both HLA-A0201 and HLA-A2402 were all matched); to allow NHF cells express the cancer testis antigen MAGEA3, and whether hTERT-specific T cells could kill the cells was observed. From the results shown in Figure 7, it could be seen that non-antigen-specific T cells had a weak killing effect (less than 10%) on both NL20 and NL20/A2.1 cells at an effector-target ratio of 2:1. The hTERT-specific T cells could kill more than 70% of NL20 and NL20/A2.1 cells at an effector-target ratio of 2:1, indicating that the specific killing effect was high. At the same time, for lung cancer cells H1299 and H1299/A2.1, non-specific T cells had nearly no killing effect at an effector-target ratio of 2:1; while the hTERT-specific T cells had a killing effect of more than 70% at an effector-target ratio of 2:1. Because H1299 cells only matched with T cells in HLA-A24, and both HLA-A0201 and HLA-A24 in H1299/A2.1 were matched with that in T cells, the T cells had a stronger killing effect on H1299/A2.1 which had a higher matching degree in HLA compared to H1299. It was suggested that the higher the matching degree of HLA, the stronger the killing ability of T cells. For NHF/MAGEA3 cells, although they expressed tumor antigen MAGEA3, the effector T cells had no obvious killing effect on them as they were specific for hTERT, suggesting that the T cells have a high killing specificity.

### Example 5. Stimulation and induction CTL, NK and NKT cells with expanded DC cells induces

One of the dilemmas faced by tumor immunotherapy is tumor immune escape. Due to the heterogeneous characteristics of tumor tissues, tumor cells in the same tissue may exhibit different immune characteristics, and the level of MHC expression may be high or low, or even not expressed at all. In this case, a single treatment with MHC-restricted killer T cells has some effects in the short term, but in the long term, it may not prevent the escape of residual immune cells, leading to tumor recurrence. Therefore, a coordinated treatment with immune cells with different killing mechanisms is a trend in future immunotherapy. A major feature of the DC cells in the DC cell resource bank provided by the present disclosure is that they can stimulate the activation and proliferation of CTL cells, and can also induce a certain proportion (25% to 90%) of CD56+ cells, i.e., NK Cells and CD3+CD56+ NKT-like cells. Using this group of mixed immune cells for tumor treatment can achieve a synergistic effect and prevent immune escape.

In this example, three DC cell lines were selected from the DC cell resource bank, and they were mixed and cultured with HLA-matched PBMCs:
A DC cell line code-named BJ3 (established after transduction with STLV3 Tax) was mixed with a collection of PBMC cells, both of which belonged to the HLA-A2 phenotype.

A DC cell line code-named BJ19 (established after transduction with STLV3 Tax) was mixed with a collection of PBMC cells, both of which belonged to the HLA-A2 phenotype.

A DC cell line code-named BJ8 (established after transduction with STLV4 Tax) was mixed with a collection of PBMC cells, both of which belonged to the HLA-A0201 and HLA-A2402 phenotypes.

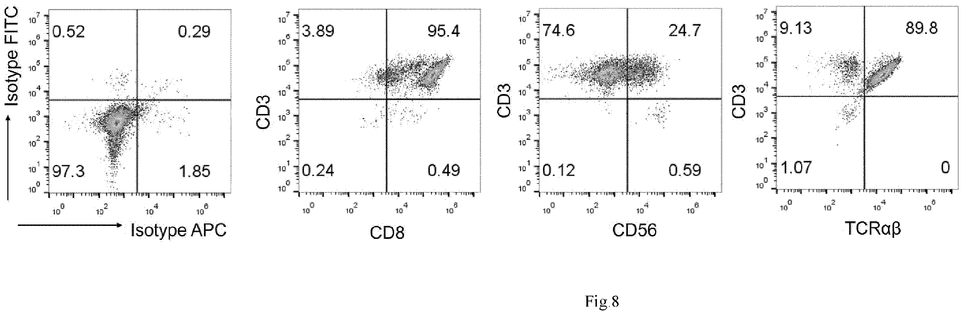

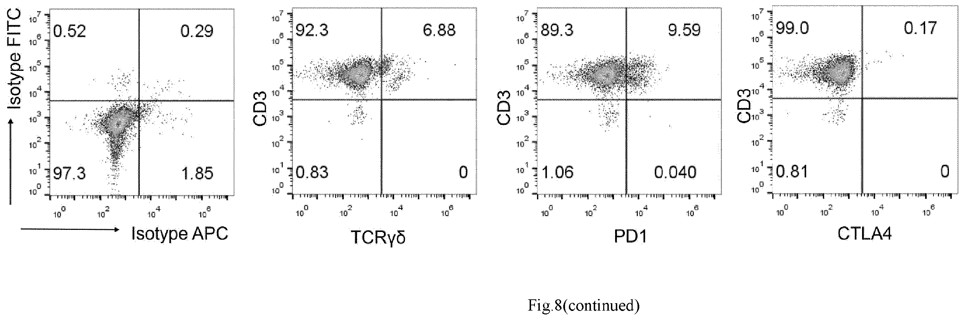

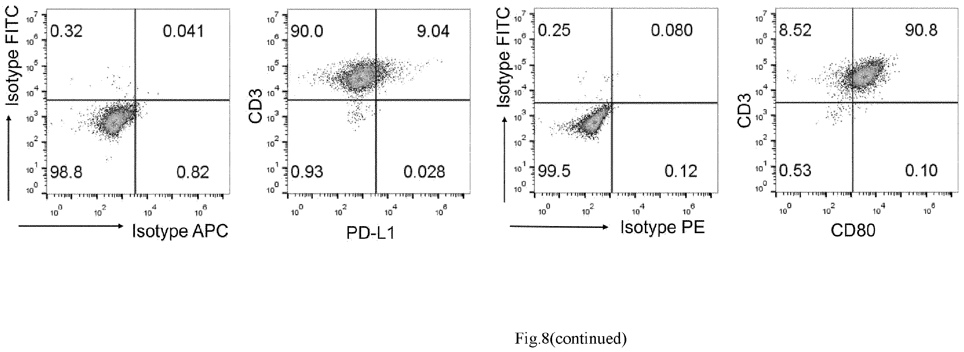

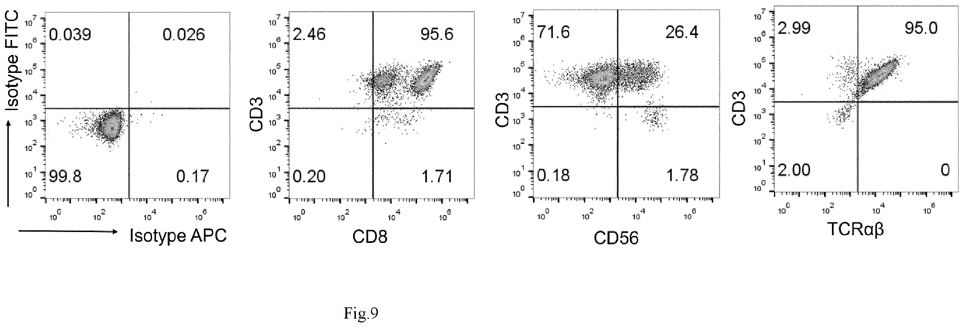

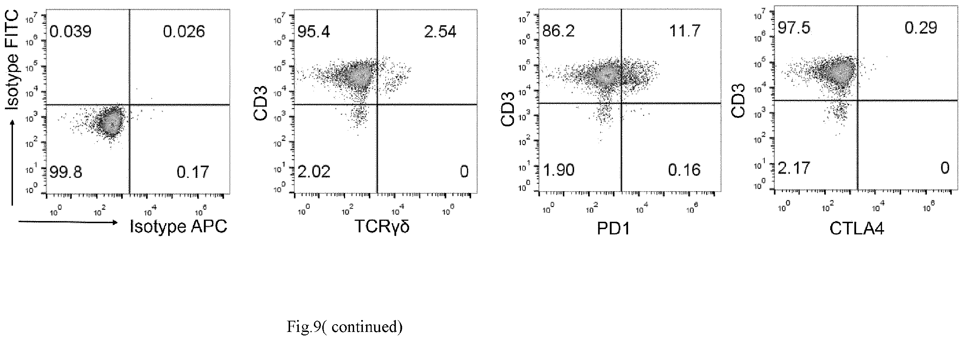

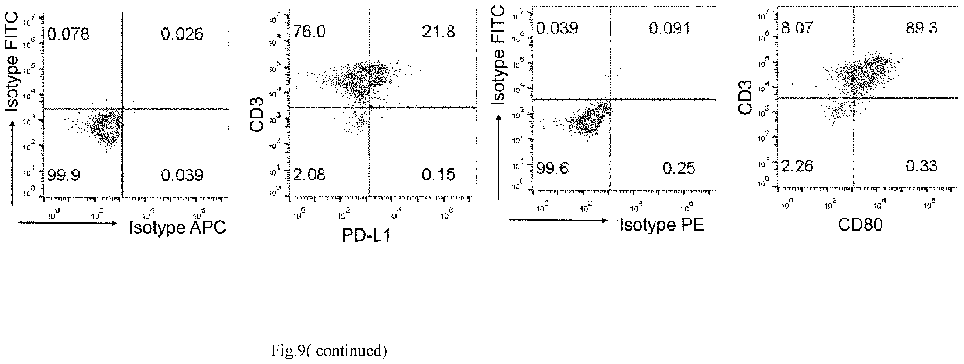

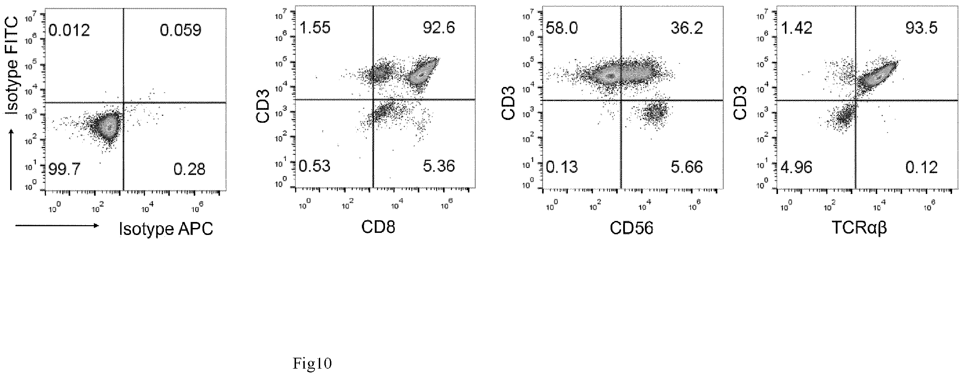

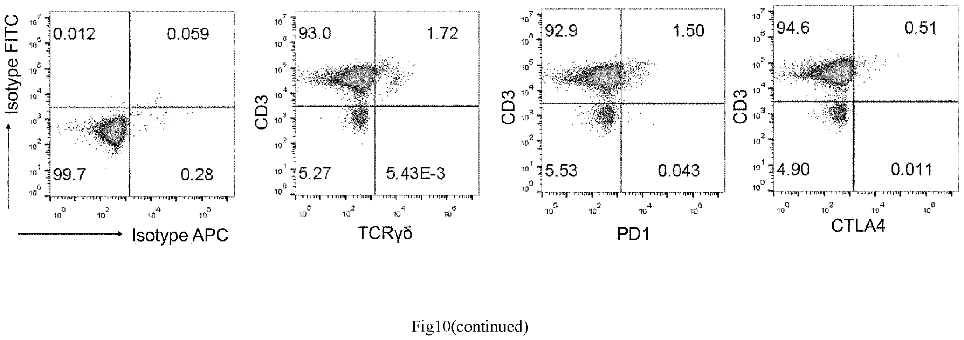

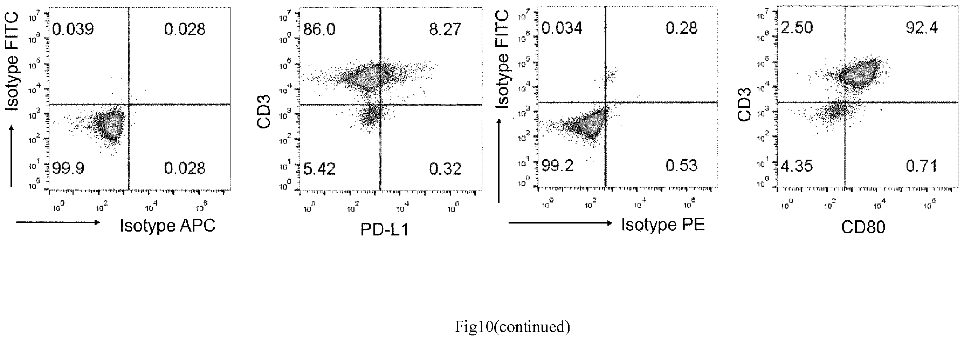

A FACS analysis was performed on the cell cultures obtained after culture, and the results were shown in Figure 8 to Figure 10, respectively. It could be seen from the figures that the cells induced by MLR test were mainly CD8+T cells, CD56+NK cells and CD3+CD56+NKT- like cells. There were two groups of CD3+CD8+ T cells. One group had bright fluorescence intensity, corresponding to relatively high expression of CD8, and the other group had weak fluorescence intensity, corresponding to relatively low expression of CD8. The induced T cells were mainly Tα/β cells, and contained only a small amount of immune suppressive molecules, such as PD1 and CTLA4.

### Example 6. Killing effect of NK cells induced by expanded DC cells on target cells

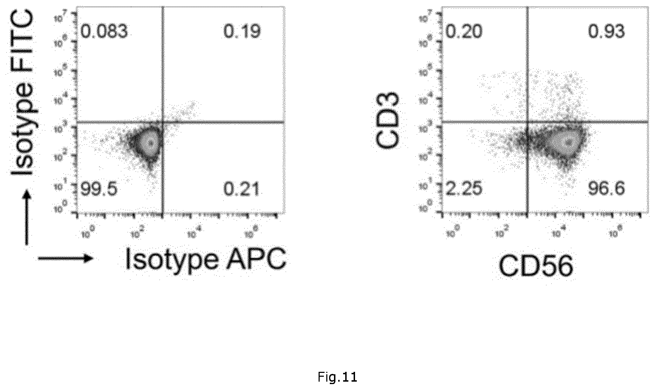

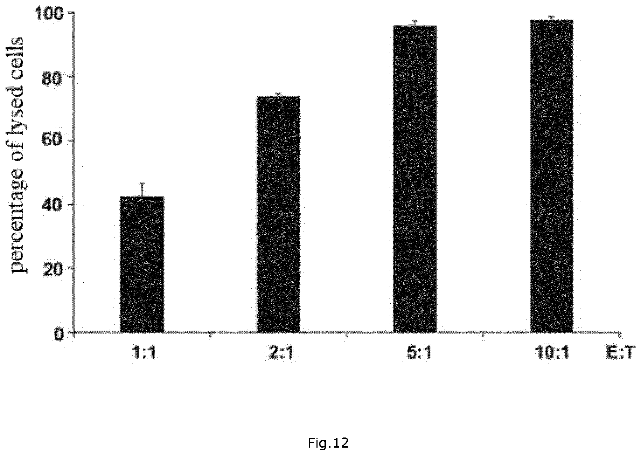

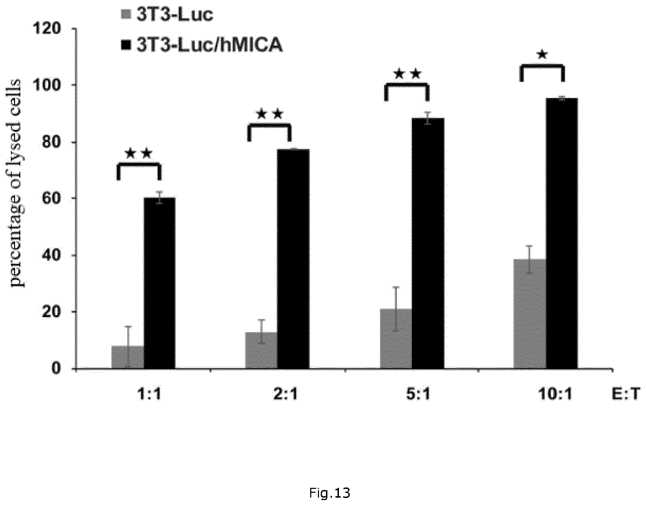

A DC cell line (BJ9) in the DC resource bank was used to induce PBMC expansion to produce two populations of CD3+ and CD3-CD56+ cells with the method described herein. After a negative selection with CD3 magnetic bead, a large number of CD3-CD56+ cells were obtained, i.e., NK cells (Figure 11). NK cells have non-MHC-restricted killing effects on tumor cells. In the study, we chose lung cancer cells A549 as the research object to observe the killing effect of the obtained NK cells on these cells. The results were shown in Figure 12. The killing effect of NK cells on A549 cells was more than 40% at an effector-target ratio 1:1. Studies have already shown that the realization of the NK cell activity mainly depends on the interaction between the activation receptor NKG2D on NK cells and the ligand MICA on the target cells. In view of the differences between mouse and human immune cells, mouse-originated cells are not sensitive to human NK cells theoretically, and are an ideal model for studying the mechanism of NK cells. In this example, mouse fibroblast cells NIH3T3 were used as the research object. They were genetically modified to express the ligand MICA, which would be determined if human NK cells would specifically kill them. The results were shown in Figure 13. 3T3-Luc cells expressed luciferase, and 3T3-Luc/hMICA cells expressed both luciferase and human MICA protein (hMICA). It could be seen from the results that human-originated NK cells had a strong and specific killing effect on murine NIH3T3 cells by recognizing the ligand MICA. For example, when the effector-target ratio was 1:1, the killing effect on 3T3-Luc/hMICA cells was as high as about 60%, while the killing effect on 3T3-Luc cells was less than 10%.

It should be pointed out that some of the examples provided herein were performed using Tax proteins from STLV3 and STLV4 viruses, but similar results were obtained using Tax proteins from viruses of other subtypes (STLV1, STLV2) (not shown).

It can be seen from the above description that the DC cell expansion method provided in the present disclosure can achieve: *in vitro* large-scale culture of primary DC cells in a cGMP environment, breaking through the number limit for therapy; ensuring that all DC cells are in a highly activated state; ensuring that DC cells are ready for genetical modification to efficiently and stably present any tumor or viral antigens as needed, achieving the characteristics of high antigen load rate and multiple-indication; effectively expanding DC cells from any individual, that is, a high efficiency of DC cell line establishment; effectively expanding a large number of DC cells via collecting only a small amount of peripheral blood or cord blood (≤ 10mL); the expanded DC cells can be permanently cryopreserved and remain their activity after resuscitation.

In terms of safety, the possibility of Tax protein of ape STLV causing human diseases has not been reported, so the risk of cross infection is extremely low. In addition, the STLV Tax protein is a foreign protein to the human body and has high immunogenicity. It can also play a function similar to that of an immune adjuvant, stimulating the activity of human immune cells and strengthening anti-tumor or anti-viral effects.

Therefore, based on the method of the present invention, we provide a human DC cell resource bank, that is, through the DC cell expansion method provided in the present invention, DC cells derived from healthy population or early-stage cancer population are engineered to realize their proliferation potential and function; at the same time, data information of each collection of DC cells (including HLA typing, molecular expression profile related to DC cell maturation and activation, *in vivo* and *in vitro* activity, etc.) are obtained. The human DC cell resource bank has great application value. First of all, it can achieve the goal of strengthening the function of autologous DC cells and serve the future treatment or prevention for cancer or viral diseases. For example, the DC resource bank accepts blood samples from healthy population or early-stage cancer patients with non-transmittable diseases, from which DC cells are extracted and activated, and stored in a deep low temperature environment after passing the quality inspection. When it is needed to use autologous DC cells for disease or prevention purposes in the future, the DC resource bank can provide a sufficient number of DC cells with potent activity. Secondly, allogeneic use of DC cells and resource sharing are realized in a way of HLA matching. All DC cells in the DC cell resource bank are screened by standard procedure and don't carry any transmittable pathogens. When cancer or acute virus infection patients cannot use their own immune cells due to low function, after HLA matching, a DC cell line suitable for the patient can be quickly found in the DC cell resource bank. The DC cell line can be thawed out, and applied to patients very quickly to save precious time for them. Third, the DC cell resource bank can provide DC cell resources for the national and even global public health and medical technology fields. It is a strategic resources for studying and conquering certain known or unknown acute viral infectious diseases.

Tax protein amino acid sequences of STLV viruses:

SEQ ID NO：1        STLV1 Tax protein

MAHFPGFGQSLLFGYPVYVFGDCVQGDWCPITGGLCSARLHRHALLATCPEHQITWDPIDGRVIGSALQFLIPRLP

SFPTQRTSKTLKVLTPPTTPKTPNIPPSFLQAMRKYSPFRNGYMEPTLGQHLPTLSFPDPGLRPQNLYTLWGGSVVC

MYLYQLSPPITWPLLPHVIFCHPGQLGAFLTNVPYKRIEELLYKISLTTGALIILPEDCLPTTLFQPARAPVTLTAWQN

GLLPFHSTLTTSGLIWTFTDGTPMISGPCPKDGQPSLVLQSSSFIFHKFQTKAYHPSFLLSHGLIQYSSFHNLHLLFE

EYANVPISLLFNEKEANDTDHEPQISPGGLEPPSEKHFRETEV

SEQ ID NO：2　　STLV2 Tax protein

MAHFPGFGQSLLYGYPVYVFGDCVQADWCPVSGGLCSTRLHRHALLATCPEHQLTWDPIDGRVVGSPLQYLIPRL

PSFPTQRTSKTLKVLTPPTTPVSPKIPPAFFQSMRKLSPYRNGCLYPTLGDQLPSLAFPDPGLRPQNIYTTWGRTVVC

LYLYQLSPPMTWPLIPHVIFCHPKQLGTFLTNVPLKRLEELLYKIFLHTGAIIVLPEDTLPTTLFQPVRAPCVQTAWDT

GLLPYHSLITTPGLIWTFNDGSPMISGPCPKPGQPSLVVQSSLLIFEKFQTKAFHPSYLLSHQLIQYSSFHNLHLLFE
EYTNIPXSYLFNEKEADDSDSDPGPSNLGAAQGESSA

SEQ ID NO：3        STLV3 Tax protein

MAHFPGFGQSLLYGYPVYVFGDCVQADWCPISGGLCSARLHRHALLATCPEHQITWDPIDGRVVSSALQYLIPRLP

SFPTQRTTRTLKVLTPPTTATTPKVPPSFFHAVRKHTPFRNNCLELTLGEQLPAMSFPDPGLRPQNVYTMWGSSVV

CLYLYQLSPPMTWPLIPHVIFCHPEQLGAFLTRVPTKRLEELLYKIFLSTGAILILPENCFPTTLFQPTRAPAIQAPWHT

GLLPCQKEITTPGLVWTFTDGSPMISGPCPKEGQPSLVVQSSTFIFQQFQTKANHPAFLLSHKLIHYSSFHSLHLLF

EEYTTVPFSLLFNEKGANVNDDEPQDEPQPPTRGQIAESSV

SEQ ID NO：4        STLV4 Tax protein

MAHFPGFGQSLLYGYPVYVFGDCVQADWCPISGGLCSPRLHRHALLATCPEHQITWDPIDGRVVGSPLQYLIPRLP

SFPTQRTSKTLKVLTPPTTPVTPKVPPSFFQSVRRHSPYRNGCLETTLGEQLPSLAFPEPGLRPQNVYTIWGKTIVCL

YIYQLSPPMTWPLIPHVIFCNPRQLGAFLSNVPPKRLEELLYKLYLHTGAIIILPEDALPTTLFQPVRAPCVQTTWNTG

LLPYQPNLTTPGLIWTFNDGSPMISGPCPKAGQPSLVVQSSLLIFERFQTKAYHPSYLLSHQLIQYSSFHHLYLLFDE

YTTIPFSLLFKEKEGDDRDNDPLPGATASPQGQN

## Claims

1. A method for expanding DC cells comprising contacting a cell sample containing DC cells to be expanded with a Tax protein derived from a simian STLV virus.

2. The method of claim 1, wherein the contacting comprises introducing an expression vector of the Tax protein into the DC cells to be expanded and allowing the Tax protein to be expressed in the DC cells to be expanded.

3. The method of claim 2, wherein the introducing is performed by lentiviral transduction.

4. The method of any one of claims 1 to 3, wherein the simian STLV virus is selected from the group consisting of STLV1, STLV2, STLV3, and STLV4 viruses.

5. The method of any one of claims 1 to 4, wherein the Tax protein comprises the amino acid sequence as set forth in SEQ ID NO: 1, 2, 3 or 4.

6. The method of any one of claims 1 to 5, wherein the cell sample is peripheral blood or cord blood.

7. The method of claim 6, wherein the DC cells to be expanded are obtained by the following steps:
a) isolating mononuclear cells from the cell sample; and
b) inducing the mononuclear cells to differentiate into DC cells.

8. The method of claim 7, wherein step b) comprises contacting the mononuclear cells with PHA and IL-2 sequentially or simultaneously, or contacting the mononuclear cells with GM-CSF and IL-4.

9. The method of any one of claims 1 to 8, further comprising introducing an expression vector of a tumor-associated antigen, a tumor-specific antigen or a viral antigen into the expanded DC cells.

10. The method of claim 9, wherein the tumor-associated antigen is hTERT.

11. The method of any one of claims 1 to 10, further comprising continuing to culture the expanded DC cells in a medium containing IL2 for 3 weeks to 2 months.

12. The method of any one of claims 1 to 11, wherein the method does not comprise the step of isolating the DC cells to be expanded from the cell sample.

13. The method of any one of claims 1 to 12, wherein the expansion success rate of the method is not less than 80%.

14. DC cells obtained by the method of any one of claims 1 to 13.

15. The DC cells of claim 14, wherein the DC cells are CD11c+ and CD205+.

16. The DC cells of claim 14 or claim 15, wherein the DC cells are CCR7+, HLA-DR+, and CD83, and/or CD40+, CD70+, 4-1BBL+, CD80+, CD83+, CD86+.

17. A method for preparing a cell culture with tumor cell or virus-infected cell killing activity from peripheral blood or cord blood mononuclear cells, comprising allowing the peripheral blood or cord blood mononuclear cells to be co-cultured with DC cells prepared by a method of any one of claims 1 to 13.

18. A cell culture prepared by the method of claim 17.

19. The cell culture of claim 18, wherein the cell culture comprises CTL cells, NK cells, and NKT cells.

20. The cell culture of claim 19, wherein the CTL cells comprise Tα/β cells and Tγ/δ cells.

21. Use of the cell culture of claim 18, 19 or 20 in the preparation of anti-tumor or anti-viral drugs.

22. A method for preparing CTL cells from peripheral blood or cord blood mononuclear cells, comprising allowing the peripheral blood or cord blood mononuclear cells to be co-cultured with DC cells prepared by a method of any one of claims 1 to 13, and isolating CD3+ cells from the co-cultured cells.

23. The method of claim 22, wherein the CTL cells comprise Tα/β cells and Tγ/δ cells.

24. A method for preparing NKT cells from peripheral blood or cord blood mononuclear cells, comprising allowing the peripheral blood or cord blood mononuclear cells to be co-cultured with DC cells prepared by a method of any one of claims 1 to 13, and isolating CD3+ and CD56+ cells from the co-cultured cells.

25. A method for preparing NK cells from peripheral blood or cord blood mononuclear cells, comprising allowing the peripheral blood or cord blood mononuclear cells to be co-cultured with DC cells prepared by a method of any one of claims 1 to 13, and isolating CD3- and CD56+ cells from the co-cultured cells.

26. A method for establishing a DC cell resource bank, comprising respectively obtaining expanded DC cells corresponding to each subject from cell samples of multiple subjects by a method of any one of claims 1 to 13.

27. The method of claim 26, wherein the number of subjects is greater than 400.

28. The method of claim 26 or 27, further comprising detecting and recording surface marker molecules of the expanded DC cells, activity of the expanded DC cells, and HLA type of the expanded DC cells.

29. The method of claim 28, wherein the detection of the activity is performed by co-cultivating the expanded DC cells and peripheral blood mononuclear cells, and detecting the killing activity of the obtained cells.

30. The method of any one of claims 26 to 29, further comprising cryopreserving the expanded DC cells corresponding to each subject separately.

31. The method of claim 30, further comprising periodically detecting and recording changes in the activity of the cryopreserved DC cells.

32. A DC cell resource bank prepared by a method of any one of claims 26 to 31.

33. A method for preparing a cell culture with tumor or virus killing activity from peripheral blood or cord blood mononuclear cells of a subject, comprising
1) detecting HLA type of the peripheral blood or cord blood mononuclear cells of the subject;
2) selecting DC cells HLA-matched with the peripheral blood or cord blood mononuclear cells of the subject from the DC cell resource bank of claim 32; and
3) co-culturing of the peripheral blood or cord blood mononuclear cells of the subject and the DC cells.


SEQUENCE LISTING

<110>  CELARTICS BIOPHARMA CO., LTD

<120>  Method for Expanding Human DC Cell and Human DC Cell Resource Library

<130>  19015CI

<150>  CN201810368646.3
<151>  2018-04-23

<160>  4

<170>  PatentIn version 3.5

<210>  1
<211>  353
<212>  PRT
<213>  Artificial Sequence

<220>
<223>  STLV1 tax

<400>  1

Met Ala His Phe Pro Gly Phe Gly Gln Ser Leu Leu Phe Gly Tyr Pro
1               5                   10                  15

Val Tyr Val Phe Gly Asp Cys Val Gln Gly Asp Trp Cys Pro Ile Thr
            20                  25                  30

Gly Gly Leu Cys Ser Ala Arg Leu His Arg His Ala Leu Leu Ala Thr
        35                  40                  45

Cys Pro Glu His Gln Ile Thr Trp Asp Pro Ile Asp Gly Arg Val Ile
    50                  55                  60

Gly Ser Ala Leu Gln Phe Leu Ile Pro Arg Leu Pro Ser Phe Pro Thr
65                  70                  75                  80

Gln Arg Thr Ser Lys Thr Leu Lys Val Leu Thr Pro Pro Thr Thr Pro
                85                  90                  95

Lys Thr Pro Asn Ile Pro Pro Ser Phe Leu Gln Ala Met Arg Lys Tyr
            100                 105                 110

Ser Pro Phe Arg Asn Gly Tyr Met Glu Pro Thr Leu Gly Gln His Leu
        115                 120                 125

Pro Thr Leu Ser Phe Pro Asp Pro Gly Leu Arg Pro Gln Asn Leu Tyr
    130                 135                 140

Thr Leu Trp Gly Gly Ser Val Val Cys Met Tyr Leu Tyr Gln Leu Ser
145                 150                 155                 160

Pro Pro Ile Thr Trp Pro Leu Leu Pro His Val Ile Phe Cys His Pro
                165                 170                 175

Gly Gln Leu Gly Ala Phe Leu Thr Asn Val Pro Tyr Lys Arg Ile Glu
            180                 185                 190

Glu Leu Leu Tyr Lys Ile Ser Leu Thr Thr Gly Ala Leu Ile Ile Leu
        195                 200                 205

Pro Glu Asp Cys Leu Pro Thr Thr Leu Phe Gln Pro Ala Arg Ala Pro
    210                 215                 220

Val Thr Leu Thr Ala Trp Gln Asn Gly Leu Leu Pro Phe His Ser Thr
225                 230                 235                 240

Leu Thr Thr Ser Gly Leu Ile Trp Thr Phe Thr Asp Gly Thr Pro Met
                245                 250                 255

Ile Ser Gly Pro Cys Pro Lys Asp Gly Gln Pro Ser Leu Val Leu Gln
            260                 265                 270

Ser Ser Ser Phe Ile Phe His Lys Phe Gln Thr Lys Ala Tyr His Pro
        275                 280                 285

Ser Phe Leu Leu Ser His Gly Leu Ile Gln Tyr Ser Ser Phe His Asn
    290                 295                 300

Leu His Leu Leu Phe Glu Glu Tyr Ala Asn Val Pro Ile Ser Leu Leu
305                 310                 315                 320

Phe Asn Glu Lys Glu Ala Asn Asp Thr Asp His Glu Pro Gln Ile Ser
                325                 330                 335

Pro Gly Gly Leu Glu Pro Pro Ser Glu Lys His Phe Arg Glu Thr Glu
            340                 345                 350

Val

<210>  2
<211>  347
<212>  PRT
<213>  Artificial Sequence

<220>
<223>  STLV2 tax

<220>
<221>  misc_feature
<222>  (317)..(317)
<223>  Xaa can be any naturally occurring amino acid

<400>  2

Met Ala His Phe Pro Gly Phe Gly Gln Ser Leu Leu Tyr Gly Tyr Pro
1               5                   10                  15

Val Tyr Val Phe Gly Asp Cys Val Gln Ala Asp Trp Cys Pro Val Ser
            20                  25                  30

Gly Gly Leu Cys Ser Thr Arg Leu His Arg His Ala Leu Leu Ala Thr
        35                  40                  45

Cys Pro Glu His Gln Leu Thr Trp Asp Pro Ile Asp Gly Arg Val Val
    50                  55                  60

Gly Ser Pro Leu Gln Tyr Leu Ile Pro Arg Leu Pro Ser Phe Pro Thr
65                  70                  75                  80

Gln Arg Thr Ser Lys Thr Leu Lys Val Leu Thr Pro Pro Thr Thr Pro
                85                  90                  95

Val Ser Pro Lys Ile Pro Pro Ala Phe Phe Gln Ser Met Arg Lys Leu
            100                 105                 110

Ser Pro Tyr Arg Asn Gly Cys Leu Tyr Pro Thr Leu Gly Asp Gln Leu
        115                 120                 125

Pro Ser Leu Ala Phe Pro Asp Pro Gly Leu Arg Pro Gln Asn Ile Tyr
    130                 135                 140

Thr Thr Trp Gly Arg Thr Val Val Cys Leu Tyr Leu Tyr Gln Leu Ser
145                 150                 155                 160

Pro Pro Met Thr Trp Pro Leu Ile Pro His Val Ile Phe Cys His Pro
                165                 170                 175

Lys Gln Leu Gly Thr Phe Leu Thr Asn Val Pro Leu Lys Arg Leu Glu
            180                 185                 190

Glu Leu Leu Tyr Lys Ile Phe Leu His Thr Gly Ala Ile Ile Val Leu
        195                 200                 205

Pro Glu Asp Thr Leu Pro Thr Thr Leu Phe Gln Pro Val Arg Ala Pro
    210                 215                 220

Cys Val Gln Thr Ala Trp Asp Thr Gly Leu Leu Pro Tyr His Ser Leu
225                 230                 235                 240

Ile Thr Thr Pro Gly Leu Ile Trp Thr Phe Asn Asp Gly Ser Pro Met
                245                 250                 255

Ile Ser Gly Pro Cys Pro Lys Pro Gly Gln Pro Ser Leu Val Val Gln
            260                 265                 270

Ser Ser Leu Leu Ile Phe Glu Lys Phe Gln Thr Lys Ala Phe His Pro
        275                 280                 285

Ser Tyr Leu Leu Ser His Gln Leu Ile Gln Tyr Ser Ser Phe His Asn
    290                 295                 300

Leu His Leu Leu Phe Glu Glu Tyr Thr Asn Ile Pro Xaa Ser Tyr Leu
305                 310                 315                 320

Phe Asn Glu Lys Glu Ala Asp Asp Ser Asp Ser Asp Pro Gly Pro Ser
                325                 330                 335

Asn Leu Gly Ala Ala Gln Gly Glu Ser Ser Ala
            340                 345

<210>  3
<211>  350
<212>  PRT
<213>  Artificial Sequence

<220>
<223>  STLV3 tax

<400>  3

Met Ala His Phe Pro Gly Phe Gly Gln Ser Leu Leu Tyr Gly Tyr Pro
1               5                   10                  15

Val Tyr Val Phe Gly Asp Cys Val Gln Ala Asp Trp Cys Pro Ile Ser
            20                  25                  30

Gly Gly Leu Cys Ser Ala Arg Leu His Arg His Ala Leu Leu Ala Thr
        35                  40                  45

Cys Pro Glu His Gln Ile Thr Trp Asp Pro Ile Asp Gly Arg Val Val
    50                  55                  60

Ser Ser Ala Leu Gln Tyr Leu Ile Pro Arg Leu Pro Ser Phe Pro Thr
65                  70                  75                  80

Gln Arg Thr Thr Arg Thr Leu Lys Val Leu Thr Pro Pro Thr Thr Ala

85                  90                  95

Thr Thr Pro Lys Val Pro Pro Ser Phe Phe His Ala Val Arg Lys His
            100                 105                 110

Thr Pro Phe Arg Asn Asn Cys Leu Glu Leu Thr Leu Gly Glu Gln Leu
        115                 120                 125

Pro Ala Met Ser Phe Pro Asp Pro Gly Leu Arg Pro Gln Asn Val Tyr
    130                 135                 140

Thr Met Trp Gly Ser Ser Val Val Cys Leu Tyr Leu Tyr Gln Leu Ser
145                 150                 155                 160

Pro Pro Met Thr Trp Pro Leu Ile Pro His Val Ile Phe Cys His Pro
                165                 170                 175

Glu Gln Leu Gly Ala Phe Leu Thr Arg Val Pro Thr Lys Arg Leu Glu
            180                 185                 190

Glu Leu Leu Tyr Lys Ile Phe Leu Ser Thr Gly Ala Ile Leu Ile Leu
        195                 200                 205

Pro Glu Asn Cys Phe Pro Thr Thr Leu Phe Gln Pro Thr Arg Ala Pro
    210                 215                 220

Ala Ile Gln Ala Pro Trp His Thr Gly Leu Leu Pro Cys Gln Lys Glu
225                 230                 235                 240

Ile Thr Thr Pro Gly Leu Val Trp Thr Phe Thr Asp Gly Ser Pro Met
                245                 250                 255

Ile Ser Gly Pro Cys Pro Lys Glu Gly Gln Pro Ser Leu Val Val Gln
            260                 265                 270

Ser Ser Thr Phe Ile Phe Gln Gln Phe Gln Thr Lys Ala Asn His Pro
        275                 280                 285

Ala Phe Leu Leu Ser His Lys Leu Ile His Tyr Ser Ser Phe His Ser
    290                 295                 300

Leu His Leu Leu Phe Glu Glu Tyr Thr Thr Val Pro Phe Ser Leu Leu
305                 310                 315                 320

Phe Asn Glu Lys Gly Ala Asn Val Asn Asp Asp Glu Pro Gln Asp Glu
                325                 330                 335

Pro Gln Pro Pro Thr Arg Gly Gln Ile Ala Glu Ser Ser Val
            340                 345                 350

<210>  4
<211>  345
<212>  PRT
<213>  Artificial Sequence

<220>
<223>  STLV4 tax

<400>  4

Met Ala His Phe Pro Gly Phe Gly Gln Ser Leu Leu Tyr Gly Tyr Pro
1               5                   10                  15

Val Tyr Val Phe Gly Asp Cys Val Gln Ala Asp Trp Cys Pro Ile Ser
            20                  25                  30

Gly Gly Leu Cys Ser Pro Arg Leu His Arg His Ala Leu Leu Ala Thr
        35                  40                  45

Cys Pro Glu His Gln Ile Thr Trp Asp Pro Ile Asp Gly Arg Val Val
    50                  55                  60

Gly Ser Pro Leu Gln Tyr Leu Ile Pro Arg Leu Pro Ser Phe Pro Thr
65                  70                  75                  80

Gln Arg Thr Ser Lys Thr Leu Lys Val Leu Thr Pro Pro Thr Thr Pro
                85                  90                  95

Val Thr Pro Lys Val Pro Pro Ser Phe Phe Gln Ser Val Arg Arg His
            100                 105                 110

Ser Pro Tyr Arg Asn Gly Cys Leu Glu Thr Thr Leu Gly Glu Gln Leu
        115                 120                 125

Pro Ser Leu Ala Phe Pro Glu Pro Gly Leu Arg Pro Gln Asn Val Tyr
    130                 135                 140

Thr Ile Trp Gly Lys Thr Ile Val Cys Leu Tyr Ile Tyr Gln Leu Ser
145                 150                 155                 160

Pro Pro Met Thr Trp Pro Leu Ile Pro His Val Ile Phe Cys Asn Pro
                165                 170                 175

Arg Gln Leu Gly Ala Phe Leu Ser Asn Val Pro Pro Lys Arg Leu Glu
            180                 185                 190

Glu Leu Leu Tyr Lys Leu Tyr Leu His Thr Gly Ala Ile Ile Ile Leu

195                 200                 205

Pro Glu Asp Ala Leu Pro Thr Thr Leu Phe Gln Pro Val Arg Ala Pro
    210                 215                 220

Cys Val Gln Thr Thr Trp Asn Thr Gly Leu Leu Pro Tyr Gln Pro Asn
225                 230                 235                 240

Leu Thr Thr Pro Gly Leu Ile Trp Thr Phe Asn Asp Gly Ser Pro Met
                245                 250                 255

Ile Ser Gly Pro Cys Pro Lys Ala Gly Gln Pro Ser Leu Val Val Gln
            260                 265                 270

Ser Ser Leu Leu Ile Phe Glu Arg Phe Gln Thr Lys Ala Tyr His Pro
        275                 280                 285

Ser Tyr Leu Leu Ser His Gln Leu Ile Gln Tyr Ser Ser Phe His His
    290                 295                 300

Leu Tyr Leu Leu Phe Asp Glu Tyr Thr Thr Ile Pro Phe Ser Leu Leu
305                 310                 315                 320

Phe Lys Glu Lys Glu Gly Asp Asp Arg Asp Asn Asp Pro Leu Pro Gly
                325                 330                 335

Ala Thr Ala Ser Pro Gln Gly Gln Asn
            340                 345